# EUROPEAN PATENT APPLICATION

(11) **EP 4 549 631 A2**
(43) Date of publication of application: **07.05.2025**
(21) Application number: 24210480.0
(22) Date of filing: 04.11.2024
(51) Int. Cl.: C25B 15/023

(54) **SHARED ELECTROLYZER MONITORING SYSTEMS AND METHODS**

(30) Priority: 06.11.2023 US 202318387380
(71) Applicant: Analog Devices, Inc., Wilmington, MA 01887 (US)
(72) Inventor: MARATHE, Radhika, Wilmington, 01887 (US); YELLEPEDDI, Atulya, Wilmington, 01887 (US)
(74) Representative: Yang, Shu

(57) **Abstract**

Described herein are electrochemical impedance spectroscopy (EIS) systems and methods that facilitate the allocation of a single stimulus generator across a number of electrolyzer stacks, each comprising a set of electrochemical cells. By distributing a stimulus signal, impedance data of the cells in each electrolyzer stack may be measured, e.g., to evaluate their condition or the condition of the stack without the need for separate stimulus blocks for each electrolyzer stack. In various embodiments, sharing the stimulus generator is enabled by sequentially directing stimulus signals to different stacks. The resulting response signals, indicative of the impedance of cells or entire stacks, are processed locally at each respective electrolyzer stack. Advantageously, the stimulus generator may be remotely located, e.g., at a location that is subject to less stringent safety measures, thereby lowering both equipment and operational expenses.

## Description

### BACKGROUND

### A. Technical Field

The present disclosure relates generally to electrolyzer systems and methods. More particularly, the present disclosure relates to a shared stimulus in EIS monitoring systems for electrolyzer measurement systems and methods for monitoring and controlling the performance of electrochemical cells within electrolyzer stacks.

### B. Background

Electrochemical Impedance Spectroscopy (EIS) monitoring systems for water electrolyzers are used to study the impedance of electrochemical cells that predominantly produce hydrogen and oxygen. Such systems are commonly used in hydrogen production intended for fuel cells and energy storage to analyze and optimize cell performance. Measuring cell impedance in any given stack of cells, typically involves superimposing a known stimulus signal on a DC signal at the operating point of the stack. A response is measured, and an impedance, which is heavily dependent on the operating point, is calculated over a range of frequencies.

The presence of electronic devices in proximity to an electrolyzer stack necessitates adherence to additional safety protocols, such as ATEX compliance, to ensure the safety of equipment operating in environments susceptible to explosion risks due to the presence of flammable gases, vapors, mists, or dust. This places additional constraints on hardware development, especially since EIS measurement devices must be placed relatively close to their respective stacks to minimize unwanted side effects such as wiring impedance and parasitics that, otherwise, would negatively affect the accuracy or resolution of the measurements. Moreover, traditional EIS methods use a dedicated stimulus generation block for each stack, which leads to high system costs as the EIS stimulus generation block tends to be among the most costly components of the hardware system.

Accordingly, what is needed are systems and methods that overcome such restrictions present in traditional designs.

### BRIEF DESCRIPTION OF THE DRAWINGS

References will be made to embodiments of the invention, examples of which may be illustrated in the accompanying figures. These figures are intended to be illustrative, not limiting. Although the invention is generally described in the context of these embodiments, it should be understood that it is not intended to limit the scope of the invention to these particular embodiments. Items in the figures are not to scale.
FIG. 1 shows a conventional EIS monitoring system.
FIG. 2 illustrates an electrolyzer system according to various embodiments of the present disclosure.
FIG. 3 is an exemplary block diagram illustrating distances for a hydrogen generation plant layout according to various embodiments of the present disclosure.
**FIG. 4** is a schematic of illustrative electrical interconnection for an exemplary system according to various embodiments of the present disclosure.
**FIG. 5** is a flowchart of an illustrative process for sharing an EIS in accordance with various embodiments of the present disclosure.

### DETAILED DESCRIPTION OF EMBODIMENTS

In the following description, for purposes of explanation, specific details are set forth in order to provide an understanding of the disclosure. It will be apparent, however, to one skilled in the art that the disclosure can be practiced without these details. Furthermore, one skilled in the art will recognize that embodiments of the present disclosure, described below, may be implemented in a variety of ways, such as a process, an apparatus, a system/device, or a method on a tangible computer-readable medium.

Components, or modules, shown in diagrams are illustrative of exemplary embodiments of the disclosure and are meant to avoid obscuring the disclosure. It shall be understood that throughout this discussion components may be described as separate functional units, which may comprise sub-units, but those skilled in the art will recognize that various components, or portions thereof, may be divided into separate components or may be integrated, including, for example, being in a single system or component. It should be noted that functions or operations discussed herein may be implemented as components. Components may be implemented in software, hardware, or a combination thereof.

Furthermore, connections between components or systems within the figures are not intended to be limited to direct connections. Rather, data between these components may be modified, re-formatted, or otherwise changed by intermediary components. Also, additional or fewer connections may be used. It shall also be noted that the terms "coupled," "connected," "communicatively coupled," "interfacing," "interface," or any of their derivatives shall be understood to include direct connections, indirect connections through one or more intermediary devices, and wireless connections. It shall also be noted that any communication, such as a signal, response, reply, acknowledgment, message, query, etc., may comprise one or more exchanges of information.

Reference in the specification to "one or more embodiments," "preferred embodiment," "an embodiment," "embodiments," or the like means that a particular feature, structure, characteristic, or function described in connection with the embodiment is included in at least one embodiment of the disclosure and may be in more than one embodiment. Also, the appearances of the above-noted phrases in various places in the specification do not necessarily all refer to the same embodiment or embodiments.

The use of certain terms in various places in the specification is for illustration and should not be construed as limiting. The terms "include," "including," "comprise," "comprising," and any of their variants shall be understood to be open terms, and any examples or lists of items are provided by way of illustration and shall not be used to limit the scope of this disclosure.

Any headings used herein are for organizational purposes only and shall not be used to limit the scope of the description or the claims. Each reference/document mentioned in this patent document is incorporated by reference herein in its entirety.

It is noted that embodiments described herein are framed in the context of shared electrochemical impedance spectroscopy (EIS) monitoring systems and methods, but one skilled in the art shall recognize that the concepts of the present disclosure are not limited to such applications and may equally be used in other contexts. For example, an alternative implementation may integrate an EIS stimulus generation block into a stack power supply.

In this document "electrochemical cell" and "electrolyzer" are used interchangeably. The terms "actuator," "contactor" and "relay" may each refer to any switching element recognized by one skilled in the art.

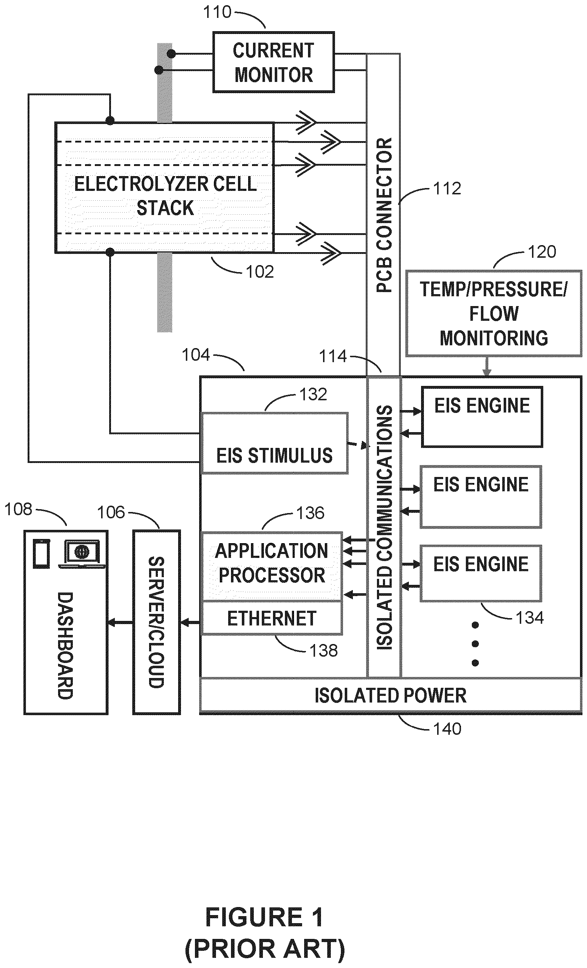

**FIG. 1** shows a conventional EIS monitoring system.

Monitoring setup 100 includes cell electrolyzer stack 102, EIS stimulus 132 (typically an AC signal generator), and a measurement system comprising EIS engines (e.g., 134) coupled to one or more cells to allow for cell-by-cell measurements. Typically, AC signal generator 132 generates, in progressively varying increments, known sinusoidal voltage or current stimulus signals of distinct frequencies and relatively small amplitudes. These stimulus signals are then applied to each electrolyzer cell in stack 102.

The measurement system examines the relationship between the stimulus signals and the resulting response signals across a specified frequency spectrum to calculate cell impedance. Response signals may be amplified and converted into an output voltage. Due to the impedance of the system, the response signals often exhibit a phase shift and amplitude changes relative to the stimulus signals. Demodulation techniques may be used to extract the amplitude and phase shift to enable separation of in-phase and out-of-phase components of the response signal to obtain complex impedance at the particular stimulus frequencies.

The collected impedance data, such as EIS spectra collected for each cell over time, may then be analyzed to gain insights into the electrochemical properties and, thus, the performance and operational conditions of individual cells within the electrolyzer stack 102. For example, by monitoring and analyzing impedance data, problems (inefficiencies, degradation, and faults in specific cells) may be detected, maintenance schedules for stacks may be optimized, and the overall efficiency and lifespan of electrolyzer stack 102 may be enhanced.

Given the relatively small amplitude of the stimulus signal, it is desirable to restrict measurement noise to achieve accurate impedance measurements. Therefore, in embodiments herein, the frequency of the sinusoidal excitation signal generated by the stimulus block is therefore confined within a relatively narrow frequency range around the frequency of interest during impedance measurements.

To further improve impedance measurement accuracy, the complex current supplied by the stimulus block may be measured simultaneously with the complex voltage across the cell or entity under test. In embodiments, to achieve this synchronization, a communications network comprising an isoSPI bus may be employed to connect the current and voltage measurements to the stimulus generator block. In embodiments, ICs measuring current and voltage may be triggered by a command that is transmitted over this communication network to perform measurements at designated frequencies.

Further, potential phase delay between the current and voltage measurement blocks, if implemented as discrete and separate units, may affect the accuracy of the impedance measurements. Therefore, in certain embodiments, the stimulus block may transmit the generated frequency with a relatively high degree of precision to measurement chips to ensure that the ICs match and measure the same frequency accurately.

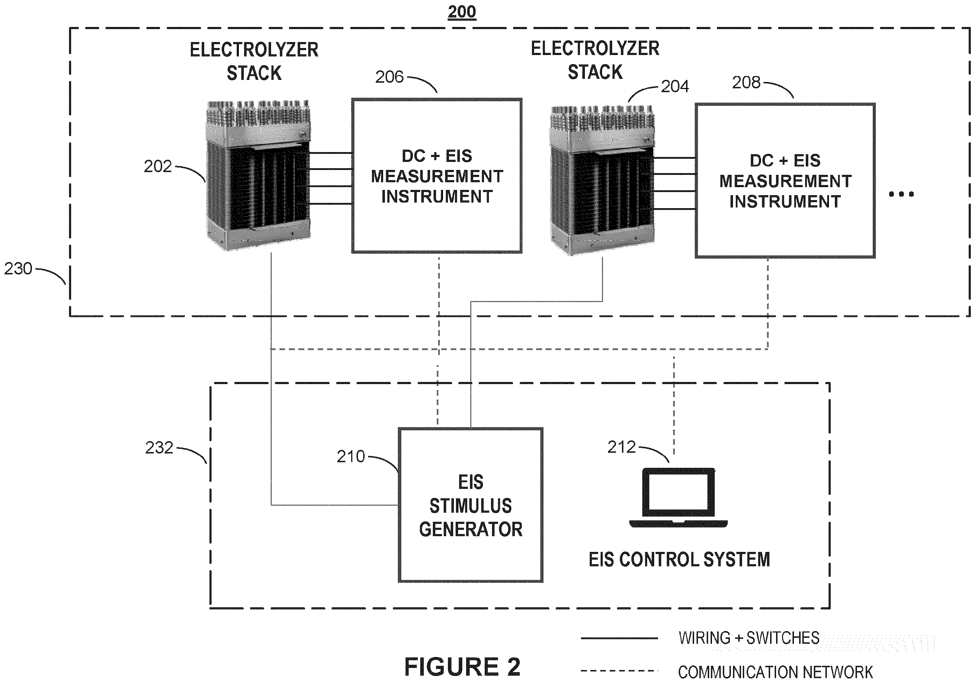

**FIG. 2** illustrates an electrolyzer system according to various embodiments of the present disclosure. In embodiments, system 200 comprises electrolyzer stacks 202, 204, measurement circuits 206, 208, EIS stimulus generator 210, and EIS control system 212. It is understood, that system 200 may comprise some or all of the elements shown in FIG. 1. It is further understood that system 200 may further comprise amplifiers, power supplies, multiplexers, switches, and other auxiliary devices not expressly shown in FIG. 2. Solid lines between elements indicate wiring and collection of switches, whereas dashed lines between elements indicate communication paths between elements. It is understood that any type of communication protocol, e.g., Ethernet or TCP IP may be employed.

As depicted in **FIG. 2****,** each measurement circuit 206, 208 is located near its corresponding electrolyzer stack 202, 204 for cell measurement purposes. In contrast, EIS stimulus generator 210, in addition to EIS control system 202, may be situated remotely from electrolyzer stacks 202, 204 and measurement units 206, 208, e.g., at location 232, which may be subject to fewer safety requirements. This arrangement allows EIS stimulus generator 210 to serve any number of electrolyzer stacks 202, 204 by sequentially applying the generated stimulus signal to different stacks 202, 204, thereby significantly reducing development, equipment, and operational costs as would be recognized by those skilled in the art.

For example, placing EIS stimulus generator 210 away from hydrogen production area 230 significantly simplifies thermal management. The EIS stimulus generator 210 can generate considerable heat (e.g., 10 kW), which can be dissipated much more effectively as cooling requirements would be less stringent to prevent unwanted contact with flammable gases, resulting in fewer design constraints and cost. Unlike in existing designs, EIS stimulus generator 210 does not require special insulation to prevent accidental exposure to hydrogen.

In operation, EIS stimulus generator 210 may generate AC voltages or currents as input stimulus signals within a specified frequency range (e.g., 0.5 kHz - 25 kHz). In embodiments, a perturbation technique may be utilized which involves generating relatively small (e.g., 1%) stimulus signals that are superimposed or modulated onto DC signals that power electrolyzer stacks 202, 204. In embodiments, the stimulus signals may be directed to any number of electrolyzer stacks (e.g., 202, 204), e.g., via a switching circuit (not shown) that performs multiplexing or switching operations, where they can be detected using any known contact or noncontact type current measurement techniques like magnetoresistive sensors. In response, each cell that receives a stimulus signal may generate an output voltage that can be detected by measurement circuits 206, 208.

Similarly, in embodiments, each measurement circuit 206, 208 may be configured to perform multiplexing or switching operations to connect the stimulus signals to different electrochemical cells or measurement points of electrolyzer stacks 202, 204, e.g., to apply the stimulus signals one at a time or to any combination of cells simultaneously. In embodiments, measurement circuits 206, 208 may be used to measure the DC signals and the stimulus signal at each electrolyzer stack 202, 204, e.g., in the form of a current, and any corresponding response signals, e.g., voltages at each electrolyzer cell in each stack 202, 204. In embodiments, to reduce noise, the frequency range of measurement circuits 206, 208 and may be limited. Further, measurement circuit 206, 208 may generate its own reference signal at a frequency that mirrors the frequency of the stimulus signal, which may have been communicated to the measurement circuit 206, 208 on a data line, e.g., to perform demodulation operations. In addition, measurement circuits 206, 208 may select a measurement time of a frequency of interest, e.g., based on a desired settling or stabilization period.

Subsequent voltage and current measurements at each frequency may be used to calculate or derive impedance data. In embodiments, control system 212 may send and receive signals to facilitate impedance measurements, e.g., periodically or according to a measurement schedule. It is understood that, in embodiments, instead of utilizing cell-by-cell impedance measurements, groups of cells may be evaluated in combination. Similarly, the impedance of a stack as a whole may be evaluated.

The impedance data may then be stored, e.g., for post-processing and data analysis by either control system 212 or a remote server implementation (not shown). Exemplary data analysis may comprise examining individual EIS spectra comparing them against each other or tracking how such spectra change over time, etc.

It is desirable to maintain parasitic impedance of the connections between electrolyzer stacks 202, 204 and their respective measurement circuits 206, 208 as short as possible, e.g., on the order of 10 µΩor less. In embodiments, this may be achieved by using lowimpedance (e.g., gold-plated) connectors and using cabling of relatively short electrical length to aid in obtaining accurate impedance measurements, which is notably challenging at low impedances. As an example, if the connector to the cell were to extend the length of the cell by about 50 cm and the thickness of the cell by about 0.5 cm, assuming a simple Cu conductor, a length of up to 1 m between the cell and the EIS measurement block may be permissible before the 10 µΩ limit is reached. Additional measures may comprise reducing noise to enhance SNR for the EIS measurement, canceling or compensating parasitic effects, and applying proper calibration protocols.

Furthermore, it is desirable to measure the input current generated by the stimulus signal and the resulting voltage measured across the cells simultaneously, such as to reduce possible phase errors that would negatively impact the measurement results.

In embodiments, where the impedance of cells in electrolyzer stack 202, 204 are on the order of tens of µΩ to a few mΩ, to accurately measure such impedance values, the cell current and the cell voltage should be measured in a synchronized manner to minimize noise. For example, to increase the accuracy of EIS measurements, the stimulus signal may be calibrated such as to provide a known waveform. In embodiments, a command serve as a trigger signal to synchronize that waveform and that measured at any given cell or combination of cells. It is understood that the calibrated the stimulus signal and/or any number of measured signals may further be processed to increase measurement accuracy. Furthermore, in embodiments, synchronization may be aided by the use of PLL circuits, clock circuits, and the like.

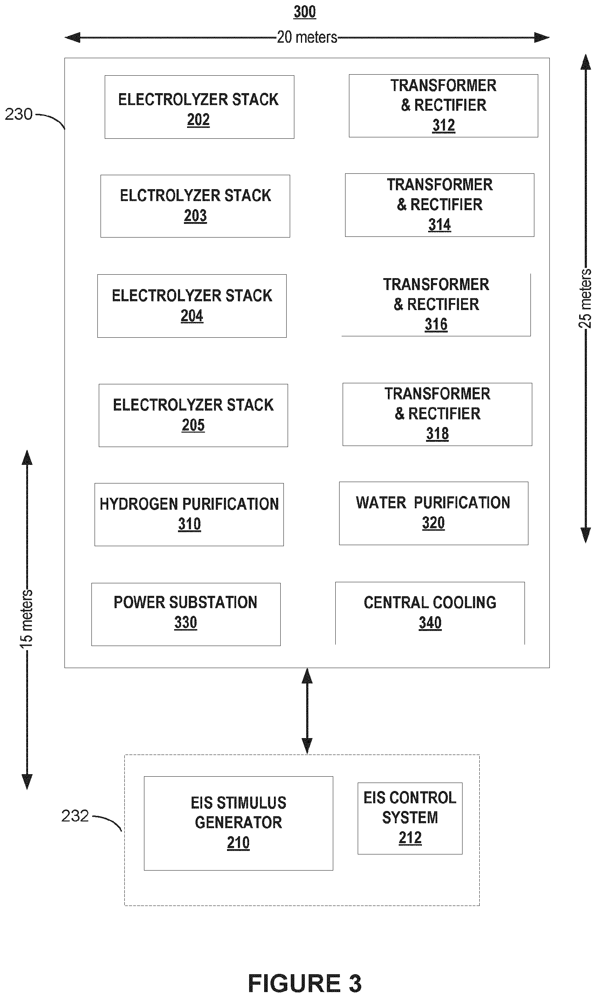

It is noted that the electrolyzer systems illustrated herein are not limited to the constructional detail shown in their respective figures or described in the accompanying text. For example, as a person of skill in the art will appreciate, a number of stacks may be used and any number of components may be modified, added or deleted. **FIG. 3** is an exemplary block diagram illustrating distances for a hydrogen generation plant layout according to various embodiments of the present disclosure. For clarity, components similar to those shown in FIG. 2 are labeled in the same manner. Items in the figures may not be to scale. Any distances and metrics in **FIG. 3** represent one exemplary embodiment depicted therein and may differ between implementations depending on the application.

In embodiments, hydrogen generation plant 300 comprises electrolyzer stacks 202-205, transformer / rectifier stations 312-318, hydrogen purification station 310, water purification station 320, power substation 130, central cooling system 340, stimulus generator 210, and EIS control system 212. For purposes of brevity, a description or their function is not repeated here. It is noted that any number of components may be modified, added, or deleted. For example, hydrogen purification system 310, which may be used to purify fuel cells within electrolyzer stacks 202-205, may be optional.

In embodiments, power substation 330 steps down a high mains voltage to medium voltage AC. Transformers and rectifiers 312-318 then convert the medium voltage AC into DC power that is provided to electrolyzer stacks 202-205, facilitating the electrolysis process to split water molecules into hydrogen and oxygen. Central cooling system 340 provides cooling water that circulates through electrolyzer stacks 202-205 to maintain electrolysis efficiency.

Unlike in existing plants, where each of electrolyzer stacks 202-205 may be assigned its dedicated stimulus generator, as depicted in **FIG. 3**, stimulus generator 210 may be located at a distance away from electrolyzer stacks 202-205, e.g., in a separate room 232 as indicated by dashed lines. This arrangement of sharing stimulus generator 210 among electrolyzer stacks 202-205, advantageously, reduces size, weight, and cost at the system level and simplifies thermal design while complying with applicable ATEX requirements.

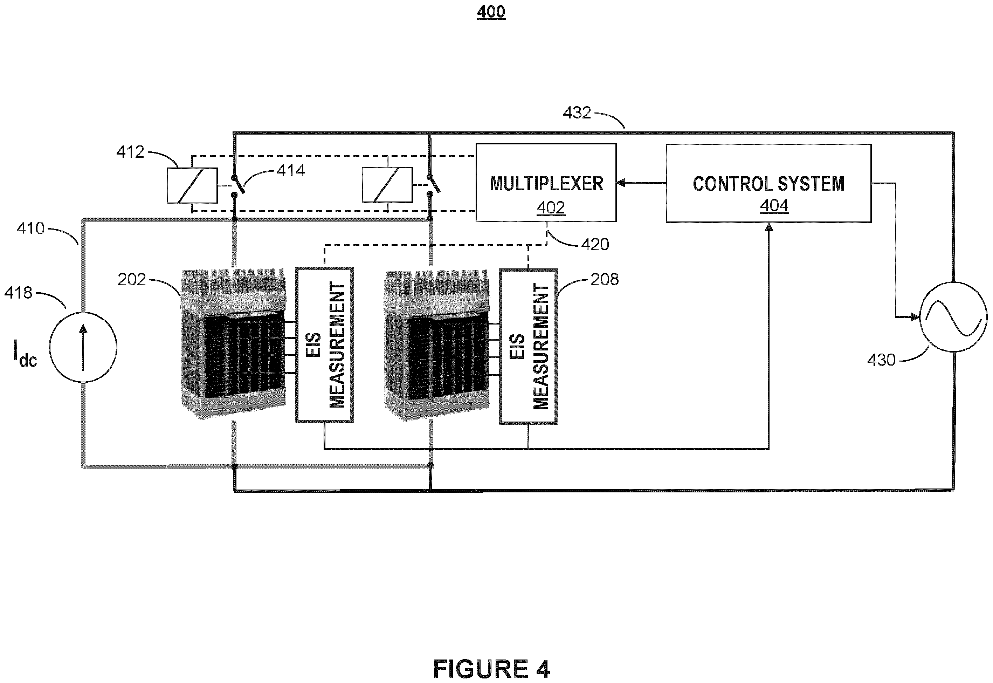

**FIG. 4** is a schematic of illustrative electrical interconnection for an exemplary system according to various embodiments of the present disclosure. The same numerals as in FIG. 2 denote similar elements. In a manner similar to FIG. 2, circuit 400 comprises electrolyzer stacks (e.g., 202), EIS measurement units (e.g., 208), multiplexor 402, control system 404, stimulus signal generator 210, and/or actuators (e.g., 412), relays (e.g., 414), and DC current source 418.

As depicted, a first signal path connection DC current source 418 to the electrolyzer stacks, providing a DC current that may be delivered over copper bus bars 410. Similarly, a high current path 432, which may be implemented as a shielded conductor, may transmit AC stimulus signals generated by stimulus signal generator 210 to the electrolyzer stacks. In addition, digital data 420 and control paths 420 may carry respective data and control signals to various parts of system 400. Furthermore, exemplary actuator 412, which may be implemented as an analog device, may be used to actuate relays 414 to enable the stimulus signal to be provided to electrolyzer stacks 202. In embodiments, actuator 412, implemented either as an analog or digital control device, may further be used to actuate EIS measurements.

In embodiments, multiplexor 402 may be coupled to actuators (e.g., 412) in high current path 432 to multiplex the stimulus signal between different electrolyzer stacks during measurement operations that may overlap with regular electrolysis operations. State differently, the stimulus signal may be superimposed on the DC current provided to electrolyzer stacks for 202, 204. Switching elements may be implemented as solid-state relays, contactors, solenoidoperated relays, and the like. It is understood that the configurations and characteristics of actuator 412, such as electrical rating (e.g., response time, voltage, current, power rating) may be selected based on the specific application. In embodiments, actuator 412 may have an AC current rating of 50-100 A at 5 kHz, and its operation may be controlled by control system 404.

As previously discussed, in embodiments, control system 404 and stimulus generator 210 may be physically distanced from the rest of system 400, separated here by high current wiring 432. Control system 404 may communicate with various components in system 402 synchronizing their operations. For example, control system 404 may instruct stimulus generator 210 to generate a signal at a certain frequency and amplitude and may further instruct multiplex of 402 which actuator to turn on at any given time. Further, control system 404 may receive measurement data from EIS measurement circuit 208 and interact with a power substation (shown FIG. 3), e.g., to determine which stack to turn on or off. It is understood that any type of communication protocol, such as Modbus (PLCs), MQTT, ControlBus, and CANBus, may be employed for these types of interactions.

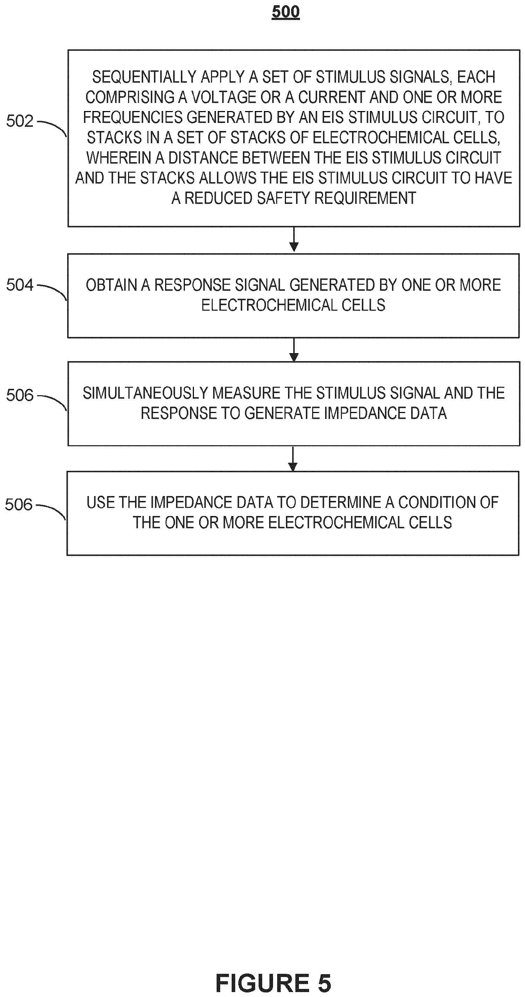

**FIG. 5** is a flowchart of an illustrative process for sharing an EIS between electrolyzer stacks in accordance with various embodiments of the present disclosure. In embodiments, sharing process 500 may start at step 502, when a number of voltage or current stimulus signals are generated by an EIS stimulus circuit. Each stimulus signal may be generated at a specific frequency. The generated stimulus signals may sequentially be applied to different stacks, each comprising a set of electrochemical cells. In embodiments, the physical distance between the EIS stimulus circuit and the stacks allows the EIS stimulus circuit to have a reduced safety requirement.

At step 504, the response signal may be obtained that has been generated by one or more of the electrochemical cells.

At step 506, both the stimulus signal and the response signal may be simultaneously measured to generate impedance data.

Finally, at 508, the impedance data may be used to determine the condition of one or more electrochemical cells.

One skilled in the art shall recognize that: (1) certain steps may optionally be performed; (2) steps may not be limited to the specific order set forth herein; (3) certain steps may be performed in different orders; and (4) certain steps may be done concurrently.

Aspects of the present invention may be encoded upon one or more non-transitory computer-readable media with instructions for one or more processors or processing units to cause steps to be performed. It shall be noted that one or more non-transitory computer-readable media shall include volatile and non-volatile memory. It shall be noted that alternative implementations are possible, including a hardware implementation or a software/hardware implementation. Hardware-implemented functions may be realized using ASIC(s), programmable arrays, digital signal processing circuitry, or the like. Accordingly, the "means" terms in any claims are intended to cover both software and hardware implementations. Similarly, the term "computer-readable medium or media" as used herein includes software and/or hardware having a program of instructions embodied thereon, or a combination thereof. With these implementation alternatives in mind, it is to be understood that the figures and accompanying description provide the functional information one skilled in the art would require to write program code (i.e., software) and/or fabricate circuits (i.e., hardware) to perform the processing required.

It shall be noted that embodiments of the present invention may further relate to computer products with a non-transitory, tangible computer-readable medium that has computer code thereon for performing various computer-implemented operations. The media and computer code may be those specially designed and constructed for the purposes of the present invention, or they may be of the kind known or available to those having skill in the relevant arts. Examples of tangible computer-readable media include, but are not limited to magnetic media such as hard disks, floppy disks, and magnetic tape; optical media such as CD-ROMs and holographic devices; magneto-optical media; and hardware devices that are specially configured to store or to store and execute program code, such as application specific integrated circuits (ASICs), programmable logic devices (PLDs), flash memory devices, and ROM and RAM devices. Examples of computer code include machine code, such as produced by a compiler, and files containing higher-level code that are executed by a computer using an interpreter. Embodiments of the present invention may be implemented in whole or in part as machine-executable instructions that may be in program modules that are executed by a processing device. Examples of program modules include libraries, programs, routines, objects, components, and data structures. In distributed computing environments, program modules may be physically located in settings that are local, remote, or both.

One skilled in the art will recognize no computing system or programming language is critical to the practice of the present disclosure. One skilled in the art will also recognize that a number of the elements described above may be physically and/or functionally separated into modules and/or sub-modules or combined.

It will be appreciated by those skilled in the art that the preceding examples and embodiments are exemplary and not limiting to the scope of the present disclosure. It is intended that all permutations, enhancements, equivalents, combinations, and improvements thereto that are apparent to those skilled in the art upon a reading of the specification and a study of the drawings are included within the true spirit and scope of the present disclosure. It shall also be noted that elements of any claims may be arranged differently including having multiple dependencies, configurations, and combinations.

### Numbered aspects

By way of non-limiting example, some aspects of the disclosure are set out in the following numbered clauses.
Numbered Clause 1. A system for sharing an electrochemical impedance spectroscopy (EIS) stimulus, the system comprising:
   an EIS stimulus circuit that generates a set of stimulus signals comprising a voltage or a current;
   a switching circuit coupled to the EIS stimulus circuit, the switching circuit applies the set of stimulus signals to stacks in a set of stacks of electrochemical cells;
   a measurement circuit coupled to the stacks, the measurement circuit simultaneously measures a stimulus signal and a response signal generated by one or more electrochemical cells to obtain impedance data; and
   a processor coupled to the measurement circuit, the processor uses the response signal to determine a condition of the one or more electrochemical cells in a stack.
Numbered Clause 2. The system according to Numbered Clause 1, wherein a distance between the EIS stimulus circuit and the stacks allows the EIS stimulus circuit to have a reduced safety requirement.
Numbered Clause 3. The system according to Numbered Clause 1 or 2, further comprising the processor analyzing the impedance data to predict conditions for the one or more electrochemical cells.
Numbered Clause 4. The system according to any preceding Numbered Clause, wherein the measurement circuit measures the impedance data within a frequency range of 5 kHz.
Numbered Clause 5. The system according to any preceding Numbered Clause, further comprising an impedance matching circuit that matches an impedance of a cells to an impedance of the measurement circuit.
Numbered Clause 6. The system according to any preceding Numbered Clause, further comprising a calibration circuit that calibrates one or more parameters of at least one of the EIS stimulus circuit or the measurement circuit.
Numbered Clause 7. The system according to any preceding Numbered Clause, further comprising an error handling circuit that uses the response signal to detect a fault or a status associated with at least some of the one or more electrochemical cells.
Numbered Clause 8. The system according to any preceding Numbered Clause, further comprising at least one of a PLL circuit or a clock circuit to synchronize the stimulus signal and the response signal.
Numbered Clause 9. The system according to any preceding Numbered Clause, wherein the measurement circuit performs steps comprising compensating a phase delay between the stimulus signal and the response signal to improve an accuracy of the impedance data.
Numbered Clause 10. A method for sharing an electrochemical impedance spectroscopy (EIS) stimulus, the method comprising:
   applying a set of stimulus signals, each comprising a voltage or a current and one or more frequencies generated by an EIS stimulus circuit, to stacks in a set of stacks of electrochemical cells, wherein a distance between the EIS stimulus circuit and the stacks allows the EIS stimulus circuit to have a reduced safety requirement;
   obtaining a response signal generated by one or more electrochemical cells; simultaneously measuring the set of stimulus signal and the response to generate impedance data; and
   using the impedance data to determine a condition of the one or more electrochemical cells.
Numbered Clause 11. The method according to Numbered Clause 10, further comprising using a demodulation technique to extract amplitude and phase shift information from the response signal to enable separation of in-phase and out-of-phase components of the response signal to obtain a complex impedance at a stimulus frequency.
Numbered Clause 12. The method according to Numbered Clause 10 or 11, further comprising analyzing the impedance data to detect at least one of an inefficiency, a degradation, or a fault associated with the one or more electrochemical cells.
Numbered Clause 13. The method according to any of Numbered Clauses 10 to 12, wherein measuring comprises measuring the impedance data within a frequency range of 5 kHz.
Numbered Clause 14. The method according to any of Numbered Clauses 10 to 13, wherein measuring comprises a perturbation method.
Numbered Clause 15. The method according to any of Numbered Clauses 10 to 14, further comprising matching an impedance of a cell to that of the measurement circuit.
Numbered Clause 16. The method according to any of Numbered Clauses 10 to 15, further comprising using a calibration circuit to calibrate one or more parameters of the EIS stimulus circuit to provide a known waveform.
Numbered Clause 17. The method according to any of Numbered Clauses 10 to 16, further comprising using the response signal to detect an error in associated with the one or more electrochemical cells.
Numbered Clause 18. The method according to any of Numbered Clauses 10 to 17, wherein the current is an alternating current of less than 100 A.
Numbered Clause 19. The method according to any of Numbered Clauses 10 to 18, wherein the measurement circuit performs steps comprising compensating a phase delay between the set of stimulus signal and the response signal to improve an accuracy of the impedance data.
Numbered Clause 20. The method according to any of Numbered Clauses 10 to 19, further comprising confining one or more frequencies of the set of stimulus signal to a relatively narrow frequency range around a frequency of interest during impedance measurements to reduce measurement noise.

## Claims

1. A system for sharing an electrochemical impedance spectroscopy (EIS) stimulus, the system comprising:
an EIS stimulus circuit that generates a set of stimulus signals comprising a voltage or a current;
a switching circuit coupled to the EIS stimulus circuit, the switching circuit applies the set of stimulus signals to stacks in a set of stacks of electrochemical cells;
a measurement circuit coupled to the stacks, the measurement circuit simultaneously measures a stimulus signal and a response signal generated by one or more electrochemical cells to obtain impedance data; and
a processor coupled to the measurement circuit, the processor uses the response signal to determine a condition of the one or more electrochemical cells in a stack.

2. The system according to claim 1, wherein a distance between the EIS stimulus circuit and the stacks allows the EIS stimulus circuit to have a reduced safety requirement.

3. The system according to claim 1 or 2, further comprising the processor analyzing the impedance data to predict conditions for the one or more electrochemical cells.

4. The system according to any preceding claim, wherein the measurement circuit measures the impedance data within a frequency range of 5 kHz.

5. The system according to any preceding claim, further comprising one or more of the following:
a. an impedance matching circuit that matches an impedance of a cells to an impedance of the measurement circuit;
b. a calibration circuit that calibrates one or more parameters of at least one of the EIS stimulus circuit or the measurement circuit;
c. an error handling circuit that uses the response signal to detect a fault or a status associated with at least some of the one or more electrochemical cells; and
d. at least one of a PLL circuit or a clock circuit to synchronize the stimulus signal and the response signal.

6. The system according to any preceding claim, wherein the measurement circuit performs steps comprising compensating a phase delay between the stimulus signal and the response signal to improve an accuracy of the impedance data.

7. A method for sharing an electrochemical impedance spectroscopy (EIS) stimulus, the method comprising:
applying a set of stimulus signals, each comprising a voltage or a current and one or more frequencies generated by an EIS stimulus circuit, to stacks in a set of stacks of electrochemical cells, wherein a distance between the EIS stimulus circuit and the stacks allows the EIS stimulus circuit to have a reduced safety requirement;
obtaining a response signal generated by one or more electrochemical cells;
simultaneously measuring the set of stimulus signal and the response to generate impedance data; and
using the impedance data to determine a condition of the one or more electrochemical cells.

8. The method according to claim 7, further comprising using a demodulation technique to extract amplitude and phase shift information from the response signal to enable separation of in-phase and out-of-phase components of the response signal to obtain a complex impedance at a stimulus frequency.

9. The method according to claim 7 or 8, further comprising analyzing the impedance data to detect at least one of an inefficiency, a degradation, or a fault associated with the one or more electrochemical cells.

10. The method according to any of claims 7 to 9, wherein measuring comprises measuring the impedance data within a frequency range of 5 kHz.

11. The method according to any of claims 7 to 10, wherein measuring comprises a perturbation method.

12. The method according to any of claims 7 to 11, further comprising one or more of the following:
a. matching an impedance of a cell to that of the measurement circuit;
b. using a calibration circuit to calibrate one or more parameters of the EIS stimulus circuit to provide a known waveform; and
c. using the response signal to detect an error in associated with the one or more electrochemical cells.

13. The method according to any of claims 7 to 12, wherein the current is an alternating current of less than 100 A.

14. The method according to any of claims 7 to 13, wherein the measurement circuit performs steps comprising compensating a phase delay between the set of stimulus signal and the response signal to improve an accuracy of the impedance data.

15. The method according to any of claims 7 to 14, further comprising confining one or more frequencies of the set of stimulus signal to a relatively narrow frequency range around a frequency of interest during impedance measurements to reduce measurement noise.
